# EUROPÄISCHE PATENTANMELDUNG

(11) **EP 2 937 683 A1**
(43) Veröffentlichungstag der Anmeldung: **28.10.2015**
(21) Anmeldenummer: 14166123.1
(22) Anmeldetag: 26.04.2014
(51) Int. Cl.: G01N 17/00, G01N 33/38

(54) **Frostbelastungsmessung mit Behinderung der Verformungen eines porösen Prüfkörpers**

(71) Anmelder: Setzer, Max J., 82152 Krailing (DE)
(72) Erfinder: Setzer, Max J., 82152 Krailing (DE)
(74) Vertreter: Dendorfer, Claus

(57) **Zusammenfassung**

Eine Vorrichtung zur Verwendung in einer Frostbelastungsmessung zur Simulation von Eigenschaften eines Bauteils (13) unter Frostbelastung weist eine Kraftaufnahmestruktur (10) auf, die mehrere Probenanker (4) und mindestens eine mit den Probenankern verbundene Gegenkraftkonstruktion (5) aufweist, wobei die mehreren Probenanker (4) dazu eingerichtet sind, in einem Prüfkörper (13) angeordnet zu werden, um frostspezifische innere Spannungen aus dem Prüfkörper (13) hin zu je einer Kraftübertragungsfläche des Prüfkörpers (13) zu leiten, und wobei die mindestens eine Gegenkraftkonstruktion (5) dazu eingerichtet ist, Kräfte aus den Probenankern (4) zu übernehmen und eine Verformung des Prüfkörpers (13) durch entsprechende Gegenkräfte zu behindern. Die Erfindung umfasst ferner ein Verfahren zum Ausführen einer Frostwiderstandsprüfung, eine Verwendung einer Kraftaufnahmestruktur, und Vorrichtungen zur Herstellung eines Prüfkörpers.

## Beschreibung

### GEBIET DER ERFINDUNG

Die vorliegende Erfindung betrifft das Gebiet der Prüfung von porösen Werkstoffen.

### HINTERGRUND DER ERFINDUNG

Ein Frosttauangriff erzeugt in einem Werkstück ein dynamisches Temperaturprofil mit damit verbundenen Spannungen. Beton und vergleichbare poröse Werkstoffe ziehen sich beim Gefrieren trotz Porenwassergehalt über die rein thermische Kontraktion hinaus zusammen (Frostschwinden), obwohl wegen der Eisbildung eine Ausdehnung erwartet wird. Durch den Frostschwind-Gefrierquellprozess in einem Frosttauwechsel wird Wasser in den Beton gesaugt (Frostsaugen). Das Frostsaugen ist der maßgebliche Parameter für die Dauerhaftigkeit des Betons unter Frosttaubelastung.

Das Frostschwinden und die Frostkräfte, die es verursachen, sind innere Kräfte des Werkstoffs, so genannte Zwangskräfte. Sie sind von Spannungen zu unterscheiden, die durch äußere Lasten und Belastungen erzeugt werden.

Die Messung von Werkstoffeigenschaften unter Frostbelastung, insbesondere die Prüfung des Frost- bzw. Frost-Tausalz-Widerstands, dient dazu, das Verhalten des Werkstoffs unter realen Bauteilbedingungen vorhersagen zu können, und um Bauteile insbesondere auf deren Dauerhaftigkeit zu bemessen. Die Lineardimensionen der Prüfkörper im Labor ist im Bereich von Dezimetern and damit klein im Verhältnis zu den realen Bauteilabmessungen z.B. von Straßen, Brücken oder Wasserbauwerken.

In älteren Prüfverfahren werden die kleinen Prüfkörper einfach im Ganzen einem Frosttauwechsel ausgesetzt. Dies ist keine adäquate Simulation der realen Bedingungen. Moderne Prüfverfahren gestalten die wärmetechnischen Bedingungen zunehmend sorgfältiger und damit näher an der Realität.

Es besteht bei allen bekannten Verfahren jedoch das Problem, dass die mechanischen Eigenschaften der Realität nicht hinreichend abgebildet werden. Es wäre daher wünschenswert, die die Zuverlässigkeit der Prognose der bekannten Verfahren auch in dieser Hinsicht zu verbessern.

### ZUSAMMENFASSUNG DER ERFINDUNG

Aufgabe der Anmeldung ist es, eine verbesserte Technik zur Frostbelastungsmessung bereitzustellen.

Erfindungsgemäß wird diese Aufgabe durch die Merkmale der unabhängigen Ansprüche gelöst. Die abhängigen Ansprüche definieren optionale Merkmale einiger Ausführungsformen der Erfindung.

Die Erfindung geht von der Grundüberlegung aus, dass ein Prüfkörper mit üblichen Abmessungen, beispielsweise im Dezimeterbereich, nur einen Ausschnitt realer Bauteile darstellt, wie z.B. von Fahrbahnen, Brücken oder Wasserbauwerken, die weit größere Dimensionen im Meterbereich haben. Das Verhalten des realen Bauteils soll in der Frostprüfung so genau wie möglich simuliert und vorhergesagt werden.

Es ist erforderlich, den Flächen des Prüfkörpers bei einem Frostangriff entsprechende Flächen realer Bauteile zuzuordnen. Sowohl bei dem realen Bauteil als auch beim Prüfkörper erfolgt die Frosteinwirkung über mindestens eine hier als Frostangriffsfläche bezeichnete Oberfläche des Bauteils. Über sie wird Wärme übertragen, aber keine Kraft. Jedoch entsprechen die anderen Oberflächen des Prüfkörpers Innenflächen des Bauteils. Über diese Innenflächen werden im realen Bauteil Kräfte übertragen, die bei der Prüfung bisher nicht erfasst werden.

Erfindungsgemäß ist vorgesehen, diese Kräfte, die durch Frost induziert werden (Zwangskräfte), beim Prüfverfahren geeignet zu berücksichtigen. Hierzu wird eine Kraftaufnahmestruktur verwendet, die über Probeanker die Frostspannungen aus dem Prüfkörper aufnimmt und zu denjenigen Oberflächen des Prüfkörpers leitet, die Innenflächen des Bauteils entsprechen. Diese Oberflächen werden hier als Kraftübertragungsflächen bezeichnet. Diese Kräfte werden von diesen Probeankern durch eine Gegenkraftkonstruktion aufgefangen. Somit wird eine Frostverformung des Prüfkörpers behindert, und damit die Behinderung der Frostverformung im realen Bauteil berücksichtigt.

In manchen Ausgestaltungen wird beachtet, dass die mindestens eine Frostangriffsfläche eine Isotherme ist, d.h. über die gesamte Frostangriffsfläche eine gleiche Temperatur hat, wenngleich diese Temperatur sich naturgemäß während eines Frost-Tau-Wechsels ändert. Im realen Bauteil ist damit der Wärmestrom definiert, der stets senkrecht zu dieser isothermen Frostangriffsfläche verläuft. In manchen Ausgestaltungen wird die Richtung der mindestens einen Kraftübertragungsfläche so gewählt, dass sie entlang der Fläche verläuft, die diesen Wärmestrom in einem Bauteil im Prüfkörper abbildet, indem sie im rechten Winkel auf die mindestens eine Frostangriffsfläche trifft. Damit die Abbildung des realen Bauteils erhalten bleibt, wird vorzugsweise die mindestens eine Kraftübertragungsfläche wärmegedämmt ausgestaltet, da auch im realen Bauteil über diese Fläche kein Wärmestrom stattfindet. Auf isothermen Flächen im Prüfkörper, die durch diese Bedingungen definiert sind, bilden sich Frostspannungen aus, die für die jeweilige Temperatur spezifisch sind. In manchen Ausgestaltungen werden die Probenanker daher im Probekörper in einer Mehrzahl von Gruppen angeordnet, wobei jede Gruppe auf einer isothermen Fläche verläuft. Damit leiten die Probenanker einer Gruppe eine spezifische, für die Temperatur charakteristische Frostspannung zu der oder den Kraftübertragungsflächen. Da die Probenanker einer Gruppe auf einer Isothermen liegen, wird über sie keine Wärme geleitet, sondern nur Kraft übertragen. Entsprechend ist die Gegenkraftkonstruktion so gestaltet, dass sie die Kräfte der Gruppe der Probenanker auf einer isothermen Ebene aufnimmt und Gegenkräfte erzeugt.

In manchen Ausgestaltungen wird eine noch präzisere Abbildung der Realität ermöglicht, indem nur eine einzige Frostangriffsfläche zugelassen wird, die eben ist und die, wie oben beschrieben, auch eine isotherme Fläche darstellt. Sie hat weiterhin 2 Symmetrieachsen, die aufeinander senkrecht stehen. Die häufigsten Varianten sind hier der Kreis und das Rechteck. Wie oben bereits beschrieben, stehen die Kraftübertragungsflächen auf dieser Frostangriffsfläche senkrecht und sind wärmegedämmt, sodass sie adiabatische Flächen im realen Bauteil abbilden. Die der Frostangriffsfläche gegenüberliegende Seite des Probekörpers ist eine Fläche, über die weder Wärme übertragen wird noch Kräfte (neutrale Fläche). Auch hier werden die Probenanker vorzugsweise auf isothermen Ebenen in Gruppen angeordnet. Die isothermen Ebenen sind bei dieser Ausgestaltung Flächen parallel zur Frostangriffsfläche. Die Probenanker einer Gruppe ordnet man vorzugsweise so an, dass sie an Punkten auf die Kraftübertragungsflächen treffen, die symmetrisch zu den 2 Symmetrieachsen der Frostangriffsfläche verlaufen. Damit ist auch die Kraftübertragung aus dem Probekörper an die Kraftübertragungsfläche symmetrisch und wegen der isothermen Anordnung auch spezifisch für die Temperatur, die sich auf dieser isothermen Fläche eingestellt hast. Die Gegenkraftkonstruktion ist so gestaltet, dass sie diese Symmetriebedingungen des Prüfkörpers aufnimmt.

In manchen Ausgestaltungen ist die Gegenkraftkonstruktion weiterentwickelt. Hier geht man davon aus, dass die symmetrischen Kräfte aus den Probenankern durch entsprechende Gegenkräfte aufgefangen werden sollen. Eine besonders zielführende Methode ist es, die Young-Laplace-Beziehung zu benutzen, gemäß der Normalkräfte, die auf einen Bogen mit definiertem Krümmungsradius treffen, im Bogen gleichmäßige Radialkräfte hervorrufen und bei konstanten Querschnitt des Bogens gleichmäßige radial Spannungen. In manchen Ausgestaltungen wird für die Gegenkraftkonstruktion ein Ringanker gewählt, der über Verbindungselemente mit den Probenankern verbunden ist. Nach der Young-Laplace-Gleichung ist hierfür zunächst ein kreisförmiger Ringanker die einfachste Lösung. Wenn er einen konstanten Querschnitt hat, dann setzt er die Normalspannungen aus den Probenankern in gleichmäßige Ringspannungen um und kompensiert so optimal die gleichmäßigen Frostspannungen auf einer isothermen Ebene. Wenn es in manchen Ausgestaltungen aus Platzgründen nicht möglich ist, einen kreisförmigen Ringanker zu nutzen, kann ein ellipsenförmiger Ringanker verwendet werden. In diesem Fall werden Normalspannungen nur dann in gleichmäßige Radialspannungen umgesetzt, wenn sich der Querschnitt entsprechend der Young-Laplace-Gleichung proportional zum Krümmungsradius der Ellipse verändert.

In manchen Ausgestaltungen wird die thermische Dämmung der Kraftübertragungsflächen allein dadurch erreicht, dass der Prüfkörper in einem eng begrenzten Raum untergebracht ist, der durch metallische Oberflächen sowohl den Strahlungsaustausch als auch die Konvektion behindert. Vorzugsweise kann ein entsprechender Austausch zwischen den Gruppen einer Vorrichtung im Außenbereich des Prüfkörpers unterdrückt werden. Dabei ist jeder Gruppe von Probenankern auf einer isothermen Ebene eine Gegenkraftkonstruktion zugeordnet. Es gilt, den Wärmeaustausch zwischen den Gegenkraftkonstruktionen zu verhindern. Dazu dient in manchen Ausgestaltungen eine Thermoschutzplatte, die zwischen jeweils zwei Gruppen angeordnet ist und so die beiden Gruppen thermisch trennt. Zur Unterdrückung der Strahlungsaustausches hat die Thermoschutzplatte vorzugsweise eine metallische Oberfläche.

In manchen Ausgestaltungen ist die mindestens eine Gegenkraftkonstruktion so bemessen, dass sie sich in Temperaturbereichen, die keiner Frostbelastungen entsprechen, thermisch gleich verformt wie der Prüfkörper. Damit wird mit ihr nur die reine Frostdehnung behindert.

In manchen Ausgestaltungen ist die mindestens eine Gegenkraftkonstruktion aus einem Material gefertigt, das bis auf die Frostdehnung eine gleiche thermische Dehnung und thermische Leitfähigkeit wie das Probenmaterial besitzt, sodass nur die reine Frostdehnung behindert wird. Ein Beispiel für ein solches Material ist ultrahochfester Beton - UHPC, Ultra High Performance Concrete, wenn der Prüfkörper aus zementgebundenen Werkstoffen besteht. Die Festigkeit dieses Materials ist vorzugsweise deutlich höher als die des Prüfkörpermaterials. Es kann beispielsweise vorgesehen sein, dass der Festigkeitsunterschied mehr als den Faktor 2 beträgt. In diesem Fall kann die Gegenkraftkonstruktion über den gesamten Prüfkörper reichen. Sie nimmt dann die Kräfte aus den einzelnen Gruppen der Probenanker aus den verschiedenen isothermen Ebenen auf. Sie behindert auch Verformungen senkrecht zu den isothermen Ebenen.

In manchen Ausgestaltungen wird ist vorgesehen, die Verformungen in der Gegenkraftkonstruktion, die durch die Kräfte aus den Probenankern erzeugt werden, zu bestimmen. Da die Temperatur in der Gegenkraftkonstruktion variabel ist, ist in manchen Ausgestaltungen vorgesehen, die rein thermische Verformung zu kompensieren. Dies geschieht vorzugsweise über Referenzpunkte, die mit der Gegenkraftkonstruktion verbunden sind, aus dem gleichen Material sind, aber ansonsten spannungsfrei bleiben.

Die Erfindung umfasst auch ein Verfahren zum Ausführen einer Frostwiderstandsprüfung.

In manchen Ausgestaltungen des Verfahrens wird eine Kraftaufnahmestruktur, die mehrere Probenanker und eine mit den Probenankern verbundene Gegenkraftkonstruktion aufweist, bereitgestellt. Ein poröser Prüfkörper, in dem die Probenanker angeordnet sind, wird bereitgestellt. Die restlichen Teile der Vorrichtung werden am Prüfkörper angebracht. Das sind beispielsweise die eine oder die mehreren Gegenkraftkonstruktionen und gegebenenfalls die Thermoschutzplatte. In manchen Ausführungsformen wird die Gegenkraftkonstruktion so befestigt, dass sie noch sicher gehalten wird aber dadurch auch keine zusätzlichen, unzulässig hohen Spannungen in den Probeankern erzeugt.

Für die Prüfung des so vorbereiteten Prüfkörpers mit der Vorrichtung wird eine in manchen Ausführungsformen eine Prüfumgebung bereitgestellt, die einen Frostangriff an der mindestens einen Frostangriffsfläche gewährleistet, und die an den Kraftübertragungsflächen entsprechende thermische Voraussetzungen schafft. Insbesondere sind in manchen Ausführungsformen die Kraftübertragungsflächen thermisch gedämmt.

Ausführungsformen des Verfahrens umfassen ferner das Durchführen eines äußeren Frostangriffs, der innere Frostspannungen im Prüfkörper und damit Verformungen des Prüfkörpers erzeugt, wobei die inneren Spannungen des Prüfkörpers durch die mehreren Probenanker nach außen geleitet und in die Gegenkraftkonstruktion eingekoppelt werden, um die Verformungen des Prüfkörpers zu behindern.

Ausführungsformen der Erfindung werden zur Prüfung poröser Werkstoffe verwendet. Charakteristisches Beispiel sind zementgebundene Werkstoffe und insbesondere Beton, der besondere Frostverformungen (Frostschwinden und Eisdehnung) aufweist.

Bestandteil der Erfindung ist ferner ein Verfahren zur Herstellung eines Prüfkörpers.

In manchen Ausgestaltungen dieses Verfahrens wird der Prüfkörper mit den Probenankern aus einem noch nicht abgebundenem, so genannten frischen Material hergestellt. Insbesondere die oben erwähnten, zementgebundenen Materialien wie Beton sind hierfür charakteristische Beispiele. Dieser Herstellungsprozess berücksichtigt vorzugsweise die Besonderheiten des Verfahrens. In manchen Ausgestaltungen wird eine Prüfkörperschalung bereitgestellt, in der der Prüfkörper aus frischen Material hergestellt werden soll. Vor dem Einbringen werden die Probenanker an präzisen Stellen positioniert und gehalten. Dazu dient vorzugsweise eine Positionierschale. In dieser Positionierschale werden mehrere Probenanker fixiert. Die Positionierschale mit den darin fixierten Probenankern wird in die Prüfkörperschalung eingebracht. Probenanker, die auf der freien Oberfläche der Probenschalung zu positionieren sind, werden über eine mindestens eine Querhalterung fixiert und positioniert, die auf der freien Oberfläche der Probekörperschalung angebracht ist.

Ferner wird in manchen Ausgestaltungen des Verfahrens in die so vorbereitete Probekörperschalung mit den Probenankern und der Positionierschale das frische Material eingebracht und verdichtet. Nach dem Erhärten wird die Querhalterung entfernt und der Probekörper mit der daran anhaftenden Positionierschale und den im Probekörper befindlichen Probenankern aus der Prüfkörperschalung entschalt.

In manchen Ausgestaltungen, in denen der Prüfkörper aus einem noch nicht abgebundenem Material hergestellt werden soll, wird für die Herstellung des späteren Prüfkörpers mit den Probenankern die Vorrichtung durch eine Positionierschale mit Fixierelementen ergänzt. Die Positionierschale besitzt in diesen Ausgestaltungen eine Mehrzahl von Fixierpunkten, die zur genauen Positionierung und Fixierung der Probenanker vorgesehen sind. An den Fixierpunkten werden die Probenanker mit dafür vorgesehenen Fixierelementen fest verbunden. Damit die Positionierschale an der Außenfläche eben bleibt, hat sie vorzugsweise Versenkungen, die ein Überstehen des Fixierelements verhindern. In manchen Ausführungsformend sind die Probenanker an der Positionierschale so befestigt, dass sie dicht sind gegenüber dem Material, das als frisches Material eingebracht werden soll. Die Positionierschale mit den Probenankern und den Fixierelementen kann so gestaltet sein, dass sie bündig in die Probekörperschalung eingesetzt werden kann.

### KURZBESCHREIBUNG DER ZEICHNUNGEN

Weitere Merkmale, Aufgaben und Vorteile der Erfindung ergeben sich aus der folgenden ausführlichen Beschreibung in Verbindung mit den beigefügten schematischen Zeichnungen, in denen zeigen:
Fig. 1a eine Seitenansicht und Fig. 1b eine Draufsicht auf eine Vorrichtung nach einem ersten Ausführungsbeispiel der Erfindung.
Fig. 2a, 2b und 2c die Vorrichtung gemäß Fig. 1a und 1b in unterschiedlichen Stufen eines Prüfverfahrens.
Fig. 3a eine Seitenansicht und Fig. 3b eine Draufsicht auf eine Vorrichtung nach einem zweiten Ausführungsbeispiel der Erfindung.
Fig. 4a, 4b und 4c die Vorrichtung gemäß Fig. 3a und 3b in unterschiedlichen Stufen eines Prüfverfahrens.
Fig. 5a - 5d die Vorrichtung nach einem dritten Ausführungsbeispiel der Erfindung; Fig. 5a und Fig. 5c sind vertikale Schnitte und Fig. 5b und Fig. 5d Draufsichten. Fig. 5a und Fig. 5b zeigen die Vorrichtung während der Herstellung mit der Probekörperschalung; Fig. 5c und Fig. 5d zeigen die Vorrichtung, wie sie für einen Frost-Tausalz-Angriff vorbereitet ist.
Fig. 6a und Fig. 6b die Vorrichtung in der Prüfkonfiguration nach einem vierten Ausführungsbeispiel, wobei Fig. 6a ein vertikale Schnitt und Fig. 6b eine Draufsicht zeigt.

### BESCHREIBUNG VON AUSFÜHRUNGSBEISPIELEN DER ERFINDUNG

### Beispiel 1: Zylinderförmige Probe - ringförmige Probenanker

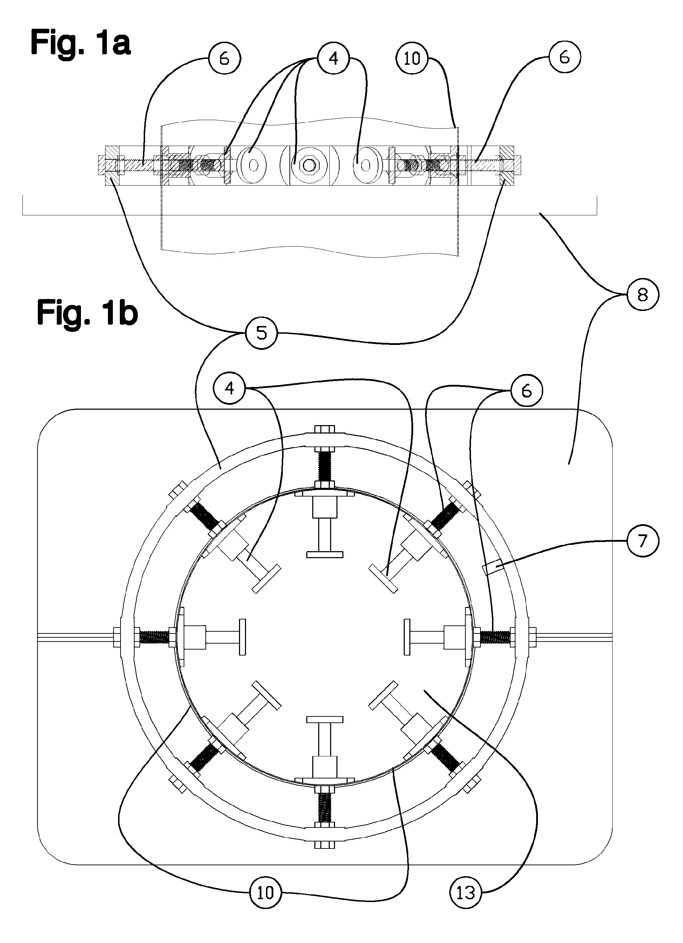

In Fig. 1 ist eine Gruppe der Vorrichtung für eine isotherme Ebene dargestellt, und zwar in der Konfiguration, die während einer Frostprüfung gegeben ist. Die Probenanker 4 - Fig. 1b - sind ringförmig angeordnet und lassen einen Kern der Probe 13 - nicht dargestellt; nur der Probenort ist gekennzeichnet - frei. Die Gegenkraftkonstruktion ist ein Ringanker 5, der mit Schrauben als Verbindungselemente 6 mit den Probenankern verbunden ist. Die Schrauben werden durch die außen liegenden Schraubenköpfe an den Ringanker gedrückt. Der Anpressdruck ist so zu gestalten, dass der Ringanker gleichmäßig und sicher gehalten wird, dass aber durch den Anpressdruck der Schrauben im Probekörper die Zugspannungen durch diesen Anpressdruck so minimiert werden, dass dieses Festhalten gerade noch erreicht wird. In der Regel wird hierzu ein Drehmomentschlüssel verwendet. Das Gegengewinde befindet sich in den jeweiligen Probenankern. Durch Gegenmuttern wird sichergestellt, dass sowohl Zug wie Druckkräfte übertragen werden können. Am Ringanker ist ein Referenzpunkt 7 befestigt. Er hat die gleiche Temperatur wie der Ringanker und ist spannungsfrei. Die Thermoschutzplatte 8 ist ebenfalls eingezeichnet. Die Positionierschale 10, an der die Probenanker fixiert sind, ist als Teilausschnitt in Fig. 1a und als Ring in Fig. 1b eingezeichnet.

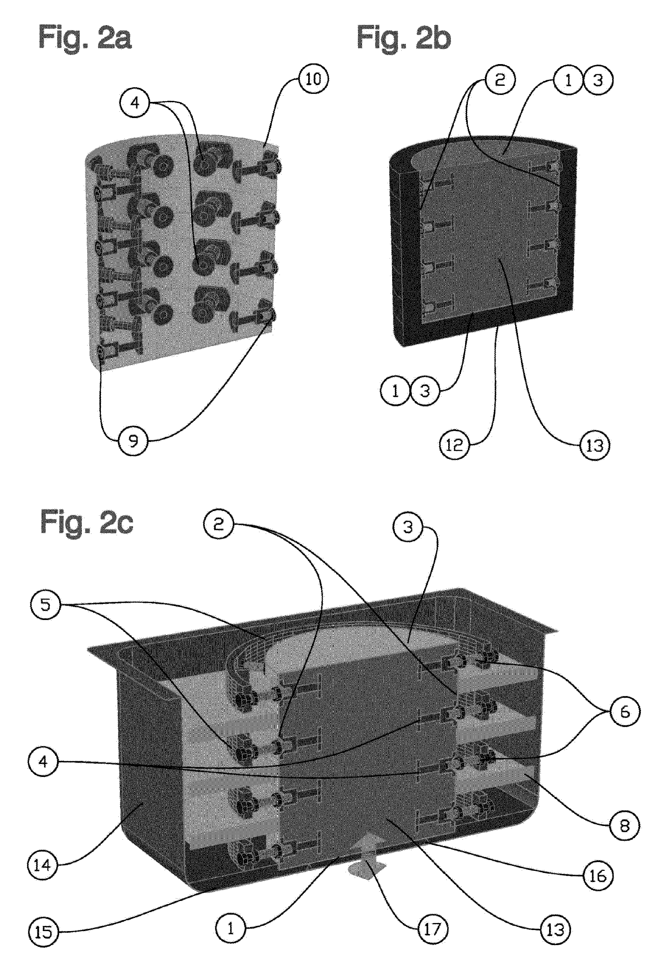

Fig. 2 zeigt die Vorrichtung und die Probe in den verschiedenen Verfahrensschritten dieses Beispiels.

Fig. 2a zeigt die Positionierschale 10 mit den Fixierschrauben 9 und den Probenankern 4, bevor sie in die Schalung zur Prüfkörperherstellung - Prüfkörperschalung 12 - eingesetzt werden und bevor die Probe hergestellt wird. Damit die Positionierschale 10 bündig in die Probekörperschalung 12 eingesetzt werden kann, muss ihre Außenfläche mit den Fixierschrauben glatt sein. Die Positionierschale 10 hat daher Versenkungen, in die die Fixierschrauben so vertieft sind, dass die Oberfläche glatt bleibt. Die Probenanker sind naturgemäß entsprechend geformt. Es ist damit die Vorrichtung bis zur Herstellung eines Prüfkörpers aus erhärtendem Material - Beton.

Fig. 2b stellt den fertigen Prüfkörper 13 in der Prüfkörperschalung 12 dar zusammen mit der Vorrichtung aus Fig. 2a. Die spätere Frostangriffsfläche 1 ist wahlweise die obere Fläche der Probe oder die Bodenseite. Die jeweils andere Seite ist dann eine neutrale Seite 3, das heißt eine Seite an der weder Wärme noch Kräfte übertragen werden. Die Kraftübertragungsflächen 2 sind in diesem Fall die Seitenflächen

Fig. 2c bildet die Konfiguration während eines Frosttauwechsel-Tests ab. Als Prüfverfahren, mit dem die Frosttauwechsel-Belastung erzeugt werden soll, entsprechend dem oben erläuterten Verfahren, und mit dem auch die thermische Dämmung der Kraftübertragungsflächen sichergestellt werden soll, ist das CIF Verfahren der RILEM Recommendation, RILEM TC 176 IDC zur Veranschaulichung gewählt. Entsprechend dem Prüfverfahren liegt hier die die Frostangriffsfläche 1 unten, der Probekörper 13 steht auf Probenständern 16 und taucht in das Prüfmedium 15 ein. Das Prüfmedium ist entweder Wasser oder Tausalzlösung. Es dient gleichzeitig als Wärmeübertragungsmedium. Dies ist möglich, weil der Testcontainer 14, in dem die gesamte Vorrichtung einschließlich dem Prüfkörper untergebracht sind, in definierter Weise in das Kältemedium einer Prüftruhe eintaucht. In der Abbildung ist der Wärmefluss 17 durch einen Doppelpfeil dargestellt. Die neutrale Fläche 3 ist die Oberfläche; die Kraftübertragungsflächen 2 sind die Seitenflächen. Es ist zu erkennen wie die Gruppen aus Probenankern 4 Verbindungselementen 6 und Ringanker 5 auf mehreren isothermen Ebenen angeordnet sind. Die Thermoschutzplatten 8 liegen zwischen den einzelnen Ebenen. Durch sie, die metallische Wand des Testcontainers und die geringen Volumina auf den jeweiligen Ebenen wird sowohl der Strahlungsaustausch als auch die Konvektion unterdrückt, so dass die Forderung nach thermischer Dämmung der Kraftübertragungsflächen sichergestellt ist.

Dieses Beispiel verdeutlicht auch den Vorteil einer zyklische Geometrie, da damit radiale Spannungen, die isothermen Flächen und der Wärmefluss besonders einfach und zielgenau realisiert und simuliert werden können.

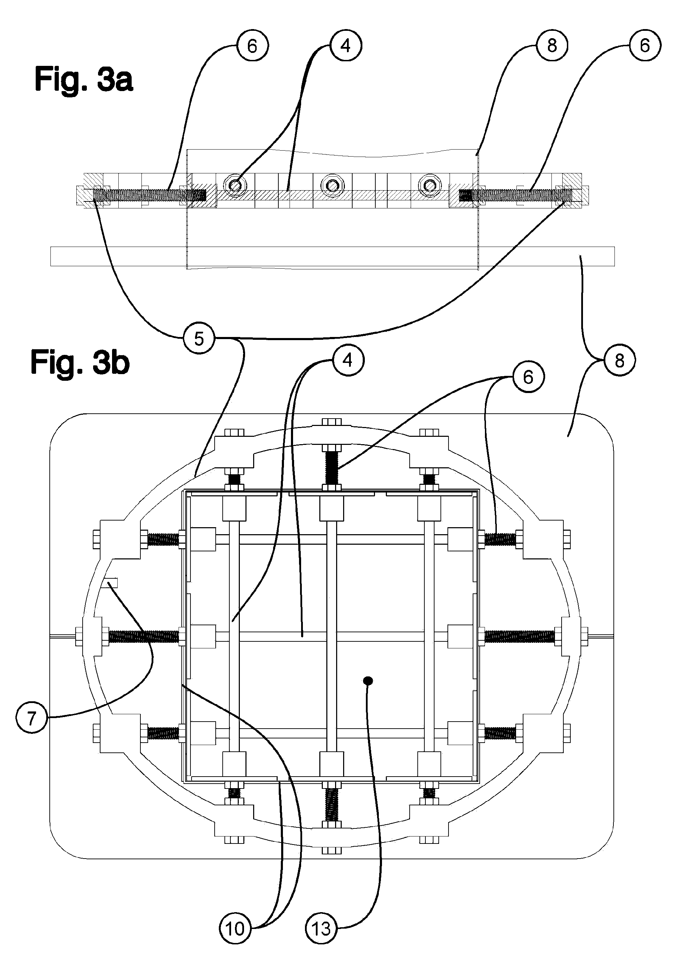

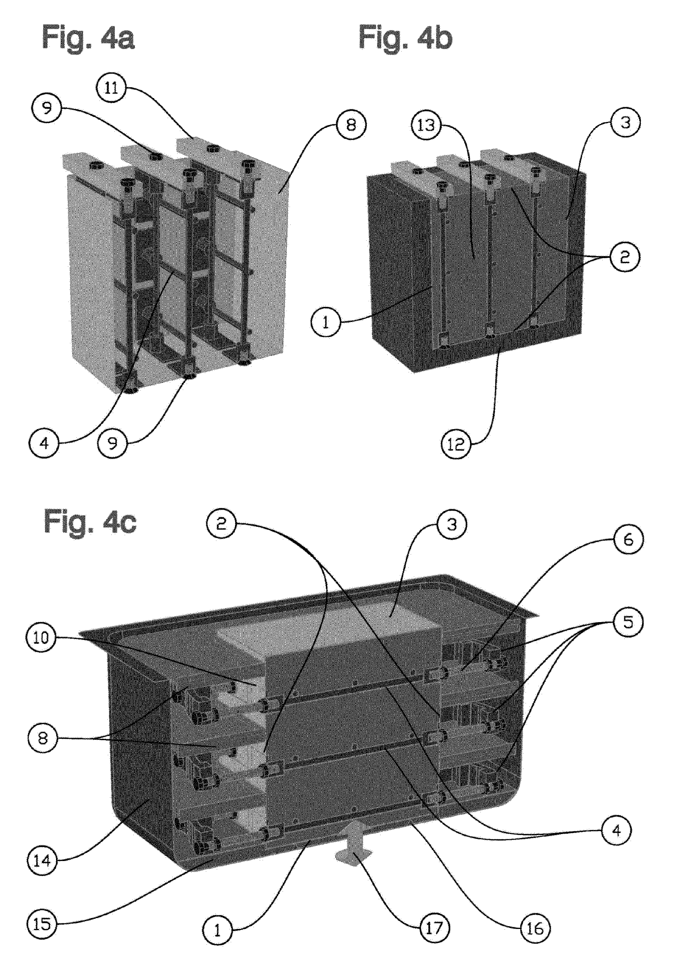

### Beispiel 2: Würfelförmiger Prüfkörper mit einer Verankerungsmatte; siehe Fig. 3 und 4.

Die Fig. 3 entspricht der Fig. 1 des Beispiels 1. Auch hier ist eine Gruppe der Vorrichtung für eine isotherme Ebene dargestellt, und zwar in der Konfiguration, die während einer Frostprüfung gegeben ist. Die Probenanker 4 - Fig. 1b - sind aber nicht ringförmig angeordnet, sondern als Bewehrungsmatte durchlaufen sie die Probe. Diese Konfiguration entspricht einem bewehrten Beton. Die übrigen Teile - Ringanker 5, Verbindungschrauben 6, Messreferenz 7, Thermoschutzplatte 8, Positionierschale 10 und Ort des Probekörpers 13 - sind äquivalent zur Fig. 1. Neben der Wahl der Probenanker 4 als Bewehrungsmatte liegt der wesentliche Unterschied in der Form des Probekörpers 13 und in der Position der Frostangriffsfläche 1.

Dies ist in Fig. 4 zu erkennen, die ansonsten der Fig. 2 des Beispiels 1 entspricht.

In diesem Beispiel wird eine Zylinderprobe des Beispiels 1 durch eine Würfelprobe ersetzt. Entscheidend ist, dass eine vertikale geschalte Fläche des Würfels die spätere Frostangriffsfläche 1 wird. Diese Methode zeigt, wie der derzeitige Stand der Technik beim CIF Test der RILEM Recommendation und in den entsprechenden EU Normen modifiziert sind. Mit der Methode von RILEM soll erreicht werden, dass eine Fläche geprüft wird, die nicht entmischt ist, wie eine Bodenfläche oder die obere, nicht geschalte Fläche eines Prüfkörpers.

In Fig. 4a ist erneut die Positionierschale 10 mit den Probenankern 4 und den Fixierschrauben 9 zu erkennen. Nachdem, wie in Fig. 4b zu sehen, diese Konfiguration in die Probekörperschalung 12 eingesetzt wird, und nachdem die Probenanker 4 senkrecht stehen, müssen sie oben durch eine gesonderte Querhalterungen 11 in ihrer Position gehalten werden.

Fig. 4b zeigt wieder den fertigen Probekörper 13 in einem Schnitt mit Probekörperschalung 12. Wie zu erkennen, liegt die Frostangriffsfläche 1 an einer geschalteten Seite. Die gegenüberliegende geschalte Seite ist die neutrale Fläche die übrigen Seitenflächen, die Bodenfläche und die Oberfläche sind Kraftübertragungsflächen 2.

In Fig. 4c ist diese Konfiguration ebenso wie in Fig. 2c in einer Prüfkonfiguration eingebaut, mit den Probenankern als Bewehrungsmatte 4, dem Ringanker 5, den Verbindungschrauben 6, der Thermoschutzplatte 8 und der Positionierschale 10. Die Testkonfiguration besteht auch hier aus dem Prüfcontainer 14, der Prüfflüssigkeit 15 und den Probekörperständern 16. Der Wärmefluss 17 ist eingezeichnet und auch hier einachsig. Auch hier ist thermische Trennung der Kraftübertragungsflächen 2 sichergestellt, wie im Beispiel 1 bereits erläutert. Die vorher geschalte Frostangriffsfläche 1 ist jetzt, und zwar ohne ein Schalenelement, die Unterseite, die neutrale Fläche 3 die Oberfläche und die Kraftübertragungsflächen 2 sind die Seitenflächen.

Das Beispiel ist charakteristisch für bewerten Beton.

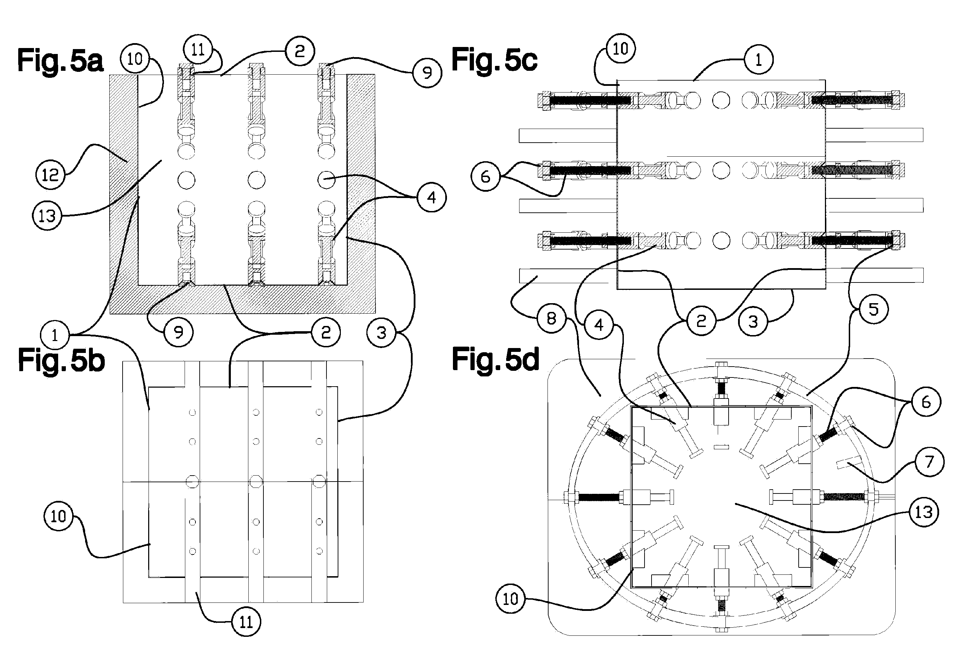

### Beispiel 3: Würfelförmiger Prüfkörper mit außen liegenden, ringförmig angeordneten Probenankern; siehe Fig. 5

Fig. 5a und Fig. 5b zeigt die Vorrichtung mit der Probekörperschalung in der Probenherstellungs-Konfiguration und Fig. 5c und 5d in der Messekonfiguration. Fig. 5a und 5c sind jeweils ein vertikaler Schnitt, und Fig. 5b und Fig. 5d sind eine Draufsicht. Anders als in Fig. 4c ist in Fig. 5c und 5d die Frostangriffsfläche 1 oben dargestellt, da hier keine spezifische Prüfmethode im Beispiel angesetzt ist.

Die verschiedenen Prüfkörperflächen sind markiert mit Frostangriffsfläche 1, Kraftübertragungsflächen 2, neutrale Fläche 3. Die Vorrichtung weist auf: Probenanker 4, Ringanker 5, Verbindungschrauben 6, Referenzpunkt 7, Thermoschutzplatte 8, Positionierschale 10, Probekörperschalung 12 und Position des Probekörpers 13.

Dieses Beispiel folgt in seiner Systematik dem Beispiel 2. Auch hier liegt die Frostangriffsfläche 1 bei der Herstellung - Fig. 5a und Fig. 5b - auf einer geschalteten Seitenfläche. Die Probenanker werden wie im Beispiel 2 durch eine Positionierschale 10 und durch Querhalterungen 11 auf der Prüfkörperschalung mit Fixierschrauben 9 gehalten. Allerdings ist die Fixierung der Probenanker, die nicht senkrecht auf die Positionierschale einbinden, etwas aufwändiger, da hier 2 Ankerpunkte für zusätzliche Fixierschrauben erforderlich sind, die nun senkrecht zur Positionierschale angeordnet sind. Dies ist erforderlich, da die Probenanker zusammen mit der fertigen Probe auch sicher entschalt werden müssen. Das heißt, dass die geschalteten Flächen glatt und eben sein müssen. Gleichzeitig müssen die Probenanker dicht aufliegen, um die Gewinde für die späteren Verbindungschrauben nicht zu verschmutzen. Die Positionierschale ist daher für diese Konfiguration ein wichtiges Bauteil.

Das Beispiel zeigt aber auch, dass mit dieser Anordnung der Probenanker die Verbindung und der Ringanker die Spannungen und Verformungen deutlich gezielter abbilden.

Das Beispiel ist charakteristisch für unbewehrter Beton.

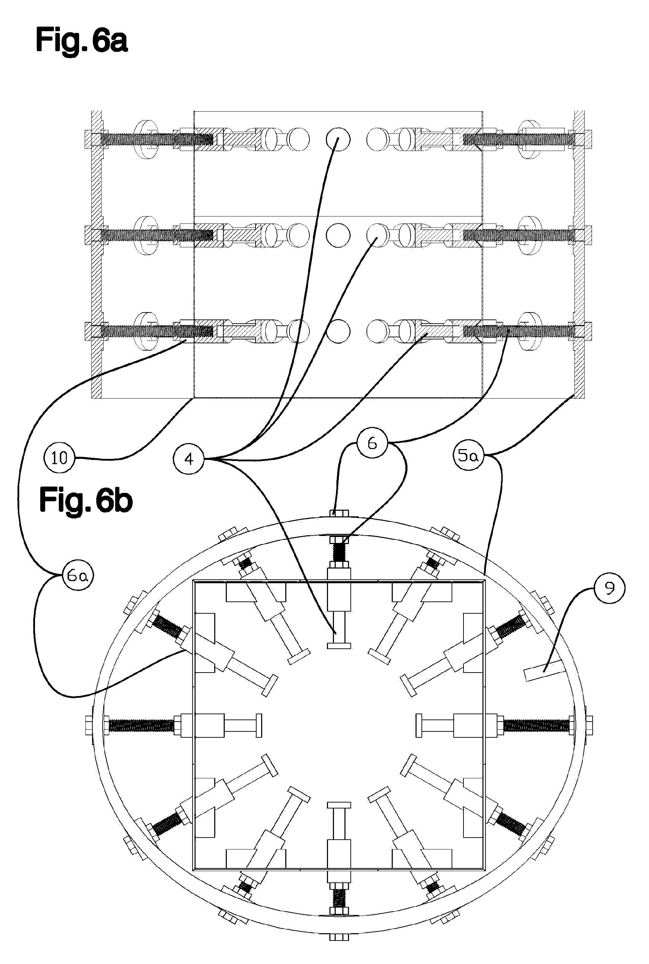

### Beispiel 4: Variante zu obigen Beispielen -Abbildung 6

Dieses Beispiel entspricht in allen Teilen der Vorrichtung dem obigen Beispiel 3 eines würfelförmiger Probekörpers mit ringförmig angeordneten Probenankern mit einem wesentlichen Unterschied: Der Ringanker 5a ist hier aus einem Material gefertigt, das die gleiche thermische Leitfähigkeit und die gleiche thermische Verformung hat wie das Material des Probekörpers. Daher kann dieser Ringanker 5a ungeteilt über die gesamte Höhe durchgehen. Durch seine thermischen Eigenschaften wird sich in ihm das gleiche Temperaturprofil wie im Probekörper einstellen und auch die gleiche thermische Dehnung bis auf die Frostdehnung. Hier können auch vertikale Kräfte übertragen werden. Bei zementgebundenen Materialien kommt als Material für den Ringanker 5a ultrahochfester Beton - UHPC Ultra High Performance Concrete - in Frage.

Die Probenanker 4 binden über die Verbindungselemente 6 auf mehreren isothermen Ebenen ein.

Für den Fall, dass das hochfeste Material nicht ausreichend ist, die Frostspannungen ausreichend aufzufangen, dann kann der Ringanker 5a noch durch die bereits oben genannten Ringanker ergänzt werden. Sie sind dann natürlich auf den jeweiligen isothermen Ebenen der beiden anderen Elemente der Bauteilgruppe angeordnet. Allerdings ist dieser Ringanker dann mit dem durchgehenden Ringanker 5a fest verbunden.

### Beispiel 5: Variante zu obigen Beispiel 4 - ohne Abbildung

Die Variante kann noch erweitert werden, wenn man den Ringanker bis zur Probenoberfläche erweitert und gleichzeitig als Einmal-Probenschalung benutzt (sog. verlorene Schalung); dies sei als Ringankerschale bezeichnet. Die Probenanker werden dann fest über die Verbindungselemente mit der Ringankerschale verbunden. Die Bodenplatte dieser verlorenen Schalung wird nach der Herstellung und dem Erhärten des Prüfkörpers entfernt, um die Bodenfläche als mögliche Frostangriffsfläche freizugeben.

### Beispiel 6

Ringanker und Verbindungselemente müssen nicht zwingend im Bereich außerhalb der Probe angeordnet sein. Vielmehr können die Probenanker direkt mit dem Ringanker fest verbunden sein und in der Probenkörperschalung bei der Herstellung positioniert werden. Sie werden dann bei der Probenherstellung zusammen mit der Probe im Probenmaterial vergossen. Diese besondere Variante einer Bauteilgruppe muss natürlich auch entsprechend auf einer isothermen Ebene angeordnet sein. Sie muss daher in geeigneter Weise, wie bereits bei den anderen Beispielen erläutert, in der Lage fixiert und positioniert sein.

Die in der obigen Beschreibung von Ausführungsbeispielen enthaltenen Einzelheiten sollen nicht als Einschränkung des Schutzbereichs der Erfindung verstanden werden, sondern als exemplarische Darstellung einiger Ausführungsformen. Viele Varianten sind möglich und dem Fachmann unmittelbar ersichtlich. Insbesondere betrifft dies Abwandlungen, die eine Kombination von Merkmalen der einzelnen Ausführungsbeispiele aufweisen. Daher soll der Bereich der Erfindung nicht durch die dargestellten Ausführungsbeispiele bestimmt werden, sondern durch die angehängten Ansprüche und ihre Äquivalente.

### NUMMERIERTE AUSFÜHRUNGSBEISPIELE

Weitere Ausführungsbeispiele der Erfindung werden nachfolgend in kompakter Form als nummerierte Ausführungsbeispiele (NA) definiert:
NA1: Verfahren zum Ausführen einer Frostwiderstandsprüfung, mit: Bereitstellen einer Kraftaufnahmestruktur, die mehrere Probenanker und eine mit den Probenankern verbundene Gegenkraftkonstruktion aufweist, Bereitstellen eines porösen Prüfkörpers, in dem die Probenanker angeordnet sind, Durchführen eines äußeren Frostangriffs, der innere Frostspannungen im Prüfkörper und damit Verformungen des Prüfkörpers erzeugt, wobei die inneren Spannungen des Prüfkörpers durch die mehreren Probenanker nach außen geleitet und in die Gegenkraftkonstruktion eingekoppelt werden, um die Verformungen des Prüfkörpers zu behindern.
NA2: Das Verfahren nach NA1 oder nach einem der Ansprüche wird angewandt auf poröse Werkstoffe, die aufgrund ihres Wassergehalts und ihrer Porenstruktur eine oder mehrere oder alle der folgenden spezifischen Eigenschaften bei Temperaturen unter 0 °C aufweisen: Ein Gefriervorgang löst zusätzlich zur normalen thermischen Dehnung spezifische innere Spannungen aus, charakterisiert durch folgende Begriffe Gefrierschwinden (Kontraktion) und Eisdehnung (Expansion); ein Tauvorgang erzeugt die dazu inversen Effekte. Unbehindert führen diese Spannungen zu zusätzlichen äußeren Verformungen, die an vorgegebenen Flächen des Prüfkörpers, den Kraftübertragungsflächen, behindert werden sollen.
NA3. Die charakteristischen Werkstoffe sind zementgebundene Werkstoffe insbesondere Beton.
NA4. Das Verfahren nach NA1 oder NA2 oder NA3 oder einem der Ansprüche wird angewandt auf Prüfkörper (13), deren Oberflächen (1,2,3) zu Flächen eines Bauteils korreliert werden. Es mindestens 2 der folgenden 3 Arten von Prüfkörperflächen unterschieden - Angriffsflächen (1), Kraftübertragungsflächen (2) und neutrale Flächen (3) - gekennzeichnet durch folgende Eigenschaften:
   - Über die Angriffsflächen erfolgt der Transport in den Prüfkörper - von Wärme, Prüfflüssigkeit und darin gelösten Stoffen. Sie bilden die Oberflächen realer Bauteile ab. Eine Kraftübertragung findet über Sie nicht statt. - Bei Wärmeübertragung werden sie im Folgenden auch Frostangriffsflächen (1) genannt, bei Massentransport Stofftransportflächen.
   - Die Kraftübertragungsflächen (2) bilden die Innenflächen einer realen Konstruktion ab. Ihre Verformungen infolge der Gefrierspannungen im Prüfkörper werden mit der Vorrichtung des Anspruchs behindert.
   - Über die neutralen Flächen (3) findet weder ein Transport von Wärme und Stoffen noch eine Kraftübertragung oder Verformungsbehinderung statt.
NA5. Die Vorrichtung, die interne Spannungen aus dem Prüfkörper (13) auf die Kraftübertragungsflächen (2) überträgt und dann die Verformung behindert, besteht aus 2 Grundelementen - den Probenankern (4) und der Gegenkraftkonstruktion, mit einer oder mehreren oder allen der folgenden Eigenschaften:
   - Die Probenanker (4) sind im Prüfkörper angeordnet und übertragen die inneren Spannungen aus dem Probenkörper zu den Kraftübertragungsflächen (2).
   - Die Gegenkraftkonstruktion (5) außerhalb der Probe nimmt die inneren Spannungen auf und behindert so die frostspezifischen Verformungen. Sie muss die Kräfte der Probenanker gleichmäßig aufnehmen.
   - Da die Probenanker regelmäßig an der Prüfkörperoberfläche enden, kann es erforderlich sein die Gegenkraftkonstruktion durch gesonderte Verbindungselemente (6) mit der Gegenkraftkonstruktion zu verbinden.
   - Die Vorrichtung kann sowohl Zug- wie Druckkräfte aus der Probe aufnehmen.
   - Die Vorrichtung ist so gestaltet und bemessen, dass sie die Frostspannungen, die bei einer bestimmten Temperatur im Prüfkörper gebildet werden, gleichmäßig nach außen überträgt und dort abfängt, und dass die Spannungen und Verformungen, die neben dieser geforderten Verformungsbehinderung durch die Vorrichtung entstehen können, minimiert werden. Probenanker und Verbindungselemente sind entsprechend angeordnet und geformt; die Ringanker entsprechend geformt und dimensioniert.
   - Die Vorrichtung wird so gestaltet und angeordnet und bemessen, dass das instationäre Temperaturprofil, das sich im Prüfkörper während eines Frostangriffs einstellt, höchstens unwesentlich verändert.
   - Durch die Verformungsbehinderung wird der Transport von Wasser und darin gelöster Stoffe im Prüfkörper während eines Frostangriffs verändert.
NA6. Die Vorrichtung soll ferner eine oder beide der folgenden thermischen Eigenschaften aufweisen:
   - Es werden nur die Verformungen unterdrückt, die auf den reinen Frostangriff zurückzuführen sind - Eisdehnung und Gefrierschwinden.
   - Ansonsten sind die thermischen Ausdehnungskoeffizienten von Vorrichtung und Prüfkörper durch entsprechende Materialwahl weitgehend gleich, so dass ohne Gefriervorgänge keine nennenswerte Differenzverformung entsteht.
NA7. Die Bauteile der Vorrichtung zur Verformungsbehinderung haben ferner eine oder mehrere oder alle der folgenden Bedingungen und Eigenschaften:
   - Die Oberflächen des Prüfkörpers zeichnen sich durch folgende besonderen Eigenschaften aus:
      -- Die Angriffsflächen (1), über die Wärme ausgetauscht wird (Frostangriffsflächen) sind isotherme Flächen (mit über die Fläche gleicher aber nicht konstanter Temperatur). Die Linien des Wärmetransports - die Adiabaten - verlaufen senkrecht zu diesen Flächen.
      -- Die Kraftübertragungsflächen (2) bilden die Adiabaten einer realen Konstruktion ab und binden daher senkrecht zu den Frostangriffsflächen ein. Ein Wärmetransport findet über Sie nicht statt. Dazu werden sie im Verfahren wärmegedämmt.
   - Die Probenanker (4) sind entlang isothermer Flächen im Prüfkörper angeordnet. Sie bilden damit die Spannungen ab, die charakteristisch für die jeweilige Temperatur unter 0 °C sind, und die sich mit der Temperatur ändern. Gleichzeitig wird ihr Einfluss auf den Wärmetransport minimiert.
   - Die Probenanker (4) auf einer isothermen Fläche bilden zusammen mit einer äußeren Gegenkraftkonstruktion (5) und den zugehörigen Verbindungselementen (6) eine Bauteilgruppe.
   - Nach den Erfordernissen eines Prüfkörpers werden mehrere solche Bauteilgruppen angeordnet.
   - Die Bauteilgruppen sind so voneinander thermisch isoliert, dass sie das Wärmeprofil, das der Prüfköper ohne sie hätte, nicht wesentlich geändert wird.
NA8. Die geometrischen Randbedingungen erfüllen eine oder mehrere oder alle der folgenden Bedingungen:
   - Es gibt eine Frostangriffsfläche.
   - Die Frostangriffsfläche (1) ist eben.
   - Der Wärmefluss verläuft damit weitgehend einachsig und senkrecht zur Frostangriffsfläche.
   - Die dazu senkrechten Seitenflächen (2) des Prüfkörpers bilden die Kraftübertragungsflächen und sind adiabatische Flächen, die wärmegedämmt sind und über die kein Stofftransport stattfindet.
   - Die der Frostangriffsfläche (3) gegenüberliegende Seite des Prüfkörpers ist so konzipiert,
   dass über Sie keine Wärme übertragen oder Kraft wird - neutrale Fläche oder Stofftransportfläche - und damit für Verfahren und Vorrichtung ohne Bedeutung ist.
NA9. Über NA8 hinaus gelten eine oder mehrere oder alle der folgenden Bedingungen:
   - Die Frostangriffsfläche (1) ist so geformt, dass sie 2 zueinander senkrechte Symmetrieachsen hat (Kreis, Rechteck).
   - Die Ebenen im Prüfkörper, die parallel zur Frostangriffsfläche verlaufen, sind isotherme Ebenen und bilden die Bezugsebenen für die Anordnung der Bauteilgruppen.
   - Die Probenanker (4) sind im Prüfkörper symmetrisch angeordnet.
   - Die Gegenkraftkonstruktion ist ein Ringanker (5). Er ist ellipsenförmig und umschließt jeweilige Gruppe der Probenanker im Prüfkörper auf einer isothermen Ebene.
   - Die Haupt- und Nebenachse der Ellipse fallen mit den Symmetrieachsen des Prüfkörpers zusammen.
   - Die Kräfte aus Probenankern mit den zugehörigen Verbindungsstücken binden an gegenüberliegenden Symmetriepunkten des ellipsenförmigen Prüfkörpers ein.
   - Die Dicke des ellipsenförmigen Ringankers (5) ändert sich proportional zum Krümmungsradius der Ellipse und gewährleistet damit nach der Young-Laplace Gleichung zusammen mit den symmetrisch eingeführten, resultierenden Normalkräften eine konstante Spannung im Ringanker.
   - Die aus den Probenankern resultierenden Tangentialkräfte werden, sofern sie eine relevante Größe überschreiten, durch einen Höhensprung des Ringankers zwischen 2 entsprechenden symmetrischen Krafteinleitungspunkten kompensiert.
NA10. Die Vorrichtung weist ferner die folgenden Eigenschaften auf: Die Thermoschutzplatte (8) trennt die Bauteilgruppen thermisch voneinander. Sie wird einer Frostbelastung im Bereich außerhalb der Probe zwischen den Bauteilgruppen eingefügt wird. Sie hat eine metallische Oberfläche, unterdrückt so den Strahlungsaustausch. Sie vermindert die Konvention durch geringe Abstände auf eine vernachlässigbare Größe.
NA11. Es gibt zwei Arten von Probenankern:
   - Probenanker (4) - "Außenbereichsprobenanker"
      -- Die Probenanker sind in einem äußeren Bereich des Prüfkörpers angeordnet.
      -- Ein innerer Kern des Prüfkörpers bleibt frei, aus dem die inneren Kräfte übertragen werden sollen.
      -- Die Probenanker nach den Regeln der Verankerungstechnik gestaltet und bemessen, wobei zusätzlich zu diesen Bemessungsregeln die unterschiedliche Verformung von Probenmaterial und Ankermaterial in Form optimierter Verhältnisse aus Elastizitätsmodul und Querschnitt minimiert wird und so in die Bemessung eingeht.
   - Probenanker (4) - "Probenankermatten" - die wie eine Bewehrungsmatte in der Probe liegen und damit auch innerhalb der Probe verformungsbehindernd sind.
NA12. Variante der Verbindungselemente (6) der Vorrichtung: Sie werden aus Schraubenverbindungen hergestellt mit entsprechenden Gegenmuttern, wobei sie mit einer präzisen Verankerungskraft gehalten werden.
NA13. Verbindungselemente (6a) nach obigem Punkt NA12 werden ergänzt durch Halterungselemente außerhalb der Probe, durch die der Halt der Gegenmuttern an dem Prüfkörper verbessert und erleichtert wird.
NA13. Ein Verformungsmesselement in der Vorrichtung misst die Verformung einer Gegenkraftkonstruktion, wobei der Einfluss der Temperaturänderung durch ein zusätzliches Messerelement (7) kompensiert wird, das auf einem Bauteil angebracht ist, das aus gleichem Material wie das Gegenkraftkonstruktion gefertigt ist, mit ihm thermisch verbunden ist aber ansonsten spannungsfrei bleibt.
NA14. Verfahren zur Herstellung von Prüfkörpern mit Fixierelementen und den folgenden Eigenschaften:
   - Der Prüfkörper (13) wird aus einem erhärtenden Material hergestellt, das im frischen Zustand in eine Probenschalung (12) eingebracht wird, in der es dann erhärtet.
   - Die Probenanker (4) werden vor der Probenherstellung in der Schalung auf geeignete Weise durch entsprechende Fixierelemente (9) der Vorrichtung so gehalten, dass die Lage der Probenanker bei der Probenherstellung unverrückt bleibt, und dass sie so angeordnet sind, wie es die Anordnung, erfordert.
   - Elemente, die zur Fixierung zusätzlich erforderlich sind, behindern ein Entschalen der Probe nicht.
NA15. Das Verfahren zur Herstellung der Prüfkörper und/oder das Prüfverfahren und/oder die Vorrichtung weist/weisen eine oder mehrere oder alle der folgenden Eigenschaften auf:
   - Der Prüfkörper wird einer symmetrischen Standardschalung (12) - Zylinderschalung, Würfelschalung - hergestellt.
   - Als Angriffsfläche (1) dient eine horizontale Fläche der so hergestellten Probe.
   - Die Kraftübertragungsflächen (2) sind die Seitenflächen.
   - Die Bauteilgruppen der Vorrichtung sind auf horizontalen Ebenen angeordnet.
   - Die Probenanker (4) an den Seitenflächen der Schalung (12) sind in der Lage mit Fixierelementen (9) gehalten.
NA16. Das Verfahren zur Herstellung der Prüfkörper und/oder das Prüfverfahren und/oder die Vorrichtung weist/weisen eine oder mehrere oder alle der folgenden Eigenschaften auf:
   - Der Prüfkörper wird in einer Würfelschalung hergestellt.
   - Als Angriffsfläche (1) dient eine vertikale Fläche der so hergestellten Probe.
   - Die Kraftübertragungsflächen (2) sind die dazu senkrechten Seitenflächen, die Bodenfläche und die Deckfläche.
   - Die Bauteilgruppen der Vorrichtung sind auf vertikalen Ebenen angeordnet.
   - Die Probenanker (4) an den Seitenflächen und er Bodenfläche der Schalung (12) sind in der Lage mit Fixierelementen (9) gehalten.
   - Die Probenanker (4), die Kräfte in Richtung der Deckfläche übertragen und deren Position durch andere Elemente nicht gesichert ist, werden durch Halterungsvorrichtungen (11) auf der Probenschalung in ihrer Lage mit Fixierelementen (9) gehalten.
NA17. Prüfkörperschalung (12) als Teil der Fixierelemente zur genauen Positionierung der Probenanker, mit den folgenden Eigenschaften:
   - Die Schalung zur Herstellung des Prüfkörpers (12) enthält Fixierpunkte für die Probenanker, an denen die Probenanker mithilfe von Fixierelementen befestigt werden.
   - Nach dem Erhärten des Prüfkörpers werden die Fixierelemente entfernt.
   - Die verbleibende Schalung mit den Fixierpunkten ist so konzipiert, dass sie ein Entschalen nicht behindert.
   - Nach dem Entschalen wird der Ringanker an den Probenankern mit den Verbindungselementen gekoppelt.
NA18. Eine Positionierschale (10) dient als Fixierelement zur genauen Anordnung der Probenanker, mit einer oder mehreren oder allen der folgenden Eigenschaften:
   - Die Positionierschale (10) ergänzt die Fixierelemente (9) an den einzelnen Probenankern, mit der die Fixierelemente (9) und Probenanker in ihrer jeweiligen Anordnung gehalten werden.
   - Die Positionierschale (10) mit den vorgegebenen Verankerungspunkten wird in die Schalung (12) bündig so eingesetzt, dass sie nach dem Erhärten des Prüfkörpers (13) zusammen mit dem Prüfkörper (13), den Probenankern (4) und den Fixierelementen (9) entschalt werden kann.
   - An den Verankerungspunkten werden die Probenanker mit Fixierelementen (9) befestigt, die nach dem Erhärten und Entschalen entfernt werden und durch die Verbindungselemente (6) ersetzt werden.
NA19. Die Positionierschale (10) weist ferner eine oder mehrere oder alle der folgenden Eigenschaften auf:
   - Die Außenfläche der Positionierschale ist metallisiert und verhindert damit einen Strahlungsaustausch.
   - Die Positionierschale behindert die Verformungseigenschaften des Prüfkörpers während eines Frostangriffs nicht.
   - Die Positionierschale kann an der Probe auch während des Frostangriffs verbleiben.
NA20. Bei der Vorrichtung sind die einzelnen Ebenen der Bauteilgruppen mechanisch verbunden aber thermisch weiterhin isoliert.
NA21. Die Gegenkraftkonstruktion (5a) in der Vorrichtung weist eine oder mehrere oder alle der folgenden Eigenschaften auf:
   - Sie ist aus einem Material hergestellt, das die gleiche thermische Leitfähigkeit und thermische Verformung besitzt wie das Material der Probe.
   - Der Ringanker mit dem gleichen Temperatur- und Verformungsprofil wie die Probe verläuft ungeteilt über die gesamte Probenhöhe.
   - Er verbindet die einzelnen Ebenen entsprechend Anspruch 0.
   - Er nimmt die Kräfte aus allen Gruppen der Probenanker und Verbindungselemente auf den verschiedenen isothermen Ebenen auf.
   - Die Festigkeit des Ankermaterials ist um ein Vielfaches (mindestens den Faktor 2 oder mindestens den Faktor 3 oder mindestens den Faktor 5) höher als das Probenmaterial.
NA22. Die Gegenkraftkonstruktion (5a) wird ferner auf den isothermen Ebenen der restlichen Bauteilgruppen aus Probenankern und Verbindungselementen durch einen zusätzlichen Ringanker ergänzt, der auf dieser Ebene zusätzlich zum Ringanker (5a) Spannungen aufnimmt, und der mit dem restlichen durchgehenden Ringanker (5a) fest verbunden ist.
NA23. Vorrichtung gemäß mindestens einem von NA1 - NA22 oder mindestens einem der Ansprüche, die ferner eine oder mehrere oder alle der folgenden Eigenschaften aufweist:
   - Gegenkraftkonstruktion (5) und Prüfkörperschalung (12) bilden eine Einheit, die Ringankerschale.
   - Sie ist aus einem Material hergestellt, das die gleiche thermische Leitfähigkeit und thermische Verformung besitzt wie das Material der Probe.
   - An der Ringankerschale sind die Probenanker über die Verbindungselemente (6) fest verbunden und in ihrer Position fixiert.
   - Die Bodenplatte der Ringankerschale ist mit der Wand, die die Probenanker trägt, so verbunden, dass sie nach dem Erhärten der Probe entfernt werden kann, und dass sie die Bodenfläche als mögliche Frostangriffsfläche frei gibt.
   - Die so gestaltete Vorrichtung ist nur für einen Prüfkörper verwendbar - verlorene Schalung.
NA24. Vorrichtung gemäß mindestens einem von NA1 - NA23 oder mindestens einem der Ansprüche, die ferner eine oder mehrere oder alle der folgenden Eigenschaften aufweist:
   - In einer Bauteilgruppe wird die Gegenkraftkonstruktion - der Ringanker (5) - mit den Probenankern (4) fest verbunden, bildet eine Einheit und schließt so die Verbindungselemente (6) mit ein.
   - Die Bauteilgruppen liegen innerhalb der Probenschalung und werden bei der Herstellung der Prüfkörper mit vergossen.
   - Diese Bauteilgruppen werden in der Probenschalung (12) in ihrer Lage fixiert.
NA25. Vorrichtung gemäß mindestens einem von NA1 - NA24 oder mindestens einem der Ansprüche, bei der die Bauteile außerhalb der Probe durch wärmedämmendes Material thermisch isoliert werden.
NA26. Vorrichtung gemäß mindestens einem von NA1 - NA25 oder mindestens einem der Ansprüche, bei der hochfestes Material für die Gegenkraftkonstruktion, den Ringanker und für die Ringankerschale verwendet wird, nämlich vorzugsweise ultrahochfester Beton - UHPC (Ultra High Performance Concrete).
NA27. Vorrichtung gemäß mindestens einem von NA1 - NA26 oder mindestens einem der Ansprüche, bei der die Probenanker auch als Messpunkte entsprechend dem Patent DE 10 2005 028 636 dienen.
NA28. Vorrichtung gemäß mindestens einem von NA1 - NA27 oder mindestens einem der Ansprüche, die dazu vorgesehen ist, an 2 Symmetriepunkten getrennt zu werden, und die es damit erlaubt, auch externe, einachsiger Lasten auf dem Prüfkörper zu übertragen.
NA29. Vorrichtung gemäß mindestens einem von NA1 - NA28 oder mindestens einem der Ansprüche, die an der Außenfläche metallische Eigenschaften hat, sodass ein Strahlungsaustausch unterdrückt wird.
NA30. Prüfverfahren gemäß mindestens einem von NA1 - NA28 oder mindestens einem der Ansprüche, bei dem die Frosttauwechselbelastung des Prüfkörpers und der Vorrichtung in einem Prüfgerät nach dem CIF Verfahren entsprechend der RILEM Recommendation durchgeführt wird.

## Patentansprüche

1. Vorrichtung zur Verwendung in einer Frostbelastungsmessung zur Simulation von Eigenschaften eines Bauteils unter Frostbelastung, wobei ein für die Frostbelastungsmessung verwendeter Prüfkörper mindestens eine Frostangriffsfläche und mindestens eine Kraftübertragungsfläche aufweist, wobei die mindestens eine Frostangriffsfläche mindestens einer Außenfläche des realen Bauteils entspricht, die der Witterung ausgesetzt ist und über die keine Kräfte übertragen werden, und die mindestens eine Kraftübertragungsfläche mindestens einer gedachten Fläche innerhalb des realen Bauteils entspricht, wobei die Vorrichtung aufweist:
- eine Kraftaufnahmestruktur, die mehrere Probenanker und mindestens eine mit den Probenankern verbundene Gegenkraftkonstruktion aufweist,
- wobei die mehreren Probenanker dazu eingerichtet sind, in dem Prüfkörper angeordnet zu werden, um frostspezifische innere Spannungen aus dem Prüfkörper hin zu je einer der Kraftübertragungsflächen des Prüfkörpers zu leiten, und
- wobei die mindestens eine Gegenkraftkonstruktion dazu eingerichtet ist, Kräfte aus den Probeankern zu übernehmen und eine Verformung des Prüfkörpers durch entsprechende Gegenkräfte zu behindern.

2. Vorrichtung nach Anspruch 1, wobei die Vorrichtung für eine Messung eingerichtet ist, bei der die mindestens eine Frostangriffsfläche des Prüfkörpers eine Isotherme bildet, und
- wobei die mindestens eine Kraftübertragungsfläche thermisch gedämmt ist,
- wobei die Probenanker eine Mehrzahl von Gruppen bilden,
- wobei die Probenanker in jeder der Gruppen so angeordnet sind, dass sie auf einer isothermen Ebene des Prüfkörpers verlaufen,
- wobei die Gegenkraftkonstruktion so gestaltet ist, dass sie die Kräfte der Gruppe der Probenanker auf einer isothermen Ebene aufnimmt und Gegenkräfte erzeugt.

3. Vorrichtung nach Anspruch 2, bei der jeder Kraftübertragungsfläche im rechten Winken auf je eine der Frostangriffsflächen trifft, und/oder bei der die isothermen Ebenen der Gruppen auch außerhalb des Prüfkörpers thermisch voneinander getrennt sind, und/oder bei der die Gegenkraftkonstruktion einer Symmetrie des Prüfkörpers folgt.

4. Vorrichtung nach Anspruch 2 oder Anspruch 3,
- wobei der Prüfkörper eine einzige, isotherme und ebene Frostangriffsfläche aufweist, die zwei zueinander senkrechte Symmetrieachsen aufweist, mit den senkrecht einbindenden Kraftübertragungsflächen einen symmetrischen Prüfkörper darstellt, und die der Frostangriffsfläche gegenüberliegende Seite thermisch und spannungsmäßig neutral ist,
- wobei die Gruppen der Probenanker auf ebenen isothermen Flächen, parallel zur Frostangriffsfläche angeordnet sind,
- wobei die Probenanker jeder Gruppe an Punkten auf die Kraftübertragungsflächen treffen, die symmetrisch zu den Symmetrieachsen der Frostangriffsfläche sind, und so symmetrisch für die Temperatur charakteristische Kräfte nach außen führen.

5. Vorrichtung nach Anspruch 4, wobei sich die symmetrische Gegenkraftkonstruktion zusammensetzt aus einem Ringanker und Verbindungselementen,
- wobei die Verbindungselemente die Kräfte aus den symmetrisch angeordneten Probenankern aufnehmen und zu dem Ringanker führen,
- wobei der Ringanker eine elliptische oder kreisförmige Form hat mit einem Querschnitt, der so bemessen ist, dass die Kräfte aus den Probenankern eine gleichmäßige Ringspannung erzeugen.

6. Vorrichtung nach einem der Ansprüche 2-5, wobei die thermische Isolation durch eine Trennung der Gegenkraftkonstruktion in einzelne Gegenkraftkonstruktionen für jede Probeankergruppe auf einer isothermen Ebene erfolgt.

7. Vorrichtung nach Anspruch 2-6, wobei zwischen je zwei Gruppen je eine Thermoschutzplatte angeordnet ist und jeder Gruppe je eine Gegenkraftkonstruktion zugeordnet ist,
- wobei jede Thermoschutzplatte eine metallische Oberfläche aufweist und so den Strahlungsaustausch unterbindet.

8. Vorrichtung nach einem der Ansprüche 1-6, wobei die mindestens eine Gegenkraftkonstruktion so bemessen wird, dass nur die Frostdehnung behindert wird, indem ihre thermische Dehnung und die thermische Dehnung des Prüfkörpers außerhalb des Frosttemperaturbereichs gleich sind.

9. Vorrichtung nach einem der Ansprüche 2-7, wobei die Gegenkraftkonstruktion so bemessen, dass sie sich in Temperaturbereichen, die keiner Frostbelastungen entsprechen, thermisch gleich verformen wie der Prüfkörper, sodass nur die reine Frostdehnung behindert wird,
- wobei die Gegenkraftkonstruktion die Kräfte aus allen Probenankern aus verschiedenen Gruppen aufnimmt,
- wobei die Gegenkraftkonstruktion auch Kräfte zwischen den Ebenen aufnimmt,
- wobei das Material der Gegenkonstruktion eine Festigkeit um mindestens einen Faktor 2 über dem Material des Prüfkörpers aufweist.

10. Vorrichtung nach einem der Ansprüche 1-9,
- wobei ein oder mehrere Referenzpunkte zur Messung einer Verformung der Gegenkraftkonstruktion so auf der Gegenkraftkonstruktion angebracht sind, dass sie die gleiche Temperatur und thermische Dehnung aufweisen wie die Stelle der Vorrichtung, deren Verformung bestimmt werden soll, aber selbst spannungsfrei sind.

11. Verfahren zum Ausführen einer Frostwiderstandsprüfung, mit:
- Bereitstellen einer Kraftaufnahmestruktur, die mehrere Probenanker und eine mit den Probenankern verbundene Gegenkraftkonstruktion aufweist,
- Bereitstellen eines porösen Prüfkörpers, in dem die Probenanker angeordnet sind, und der zur Verwendung in einer Frostbelastungsmessung zur Simulation von Eigenschaften eines Bauteils vorgesehen ist,
- Durchführen eines äußeren Frostangriffs, der innere Frostspannungen im Prüfkörper und damit Verformungen des Prüfkörpers erzeugt, wobei die inneren Spannungen des Prüfkörpers durch die mehreren Probenanker nach außen geleitet und in die Gegenkraftkonstruktion eingekoppelt werden, um die Verformungen des Prüfkörpers zu behindern.

12. Verwendung einer Kraftaufnahmestruktur mit einer Gegenkraftkonstruktion und mehreren damit verbundenen Probenankern zur Ausführung eines Verfahrens nach Anspruch 11, wobei das Material des Prüfkörpers ein zementgebundener Werkstoff ist.

13. Verfahren zur Herstellung eines Prüfkörpers, der zur Verwendung in einer Frostbelastungsmessung zur Simulation von Eigenschaften eines Bauteils vorgesehen ist, insbesondere zum Ausführen einer Frostwiderstandsprüfung nach Anspruch 11, mit:
- Bereitstellen einer äußeren Prüfkörperschalung,
- Fixieren mehrerer Probenanker in einer Positionierschale,
- Einbringen der Positionierschale mit den darin fixierten Probenankern in die Prüfkörperschalung,
- Einbringen von frischem Material für den Prüfkörper in die Prüfkörperschalung,
- Verdichten des Materials in der Prüfkörperschalung,
- Entschalen des Prüfkörpers mit der daran anhaftenden Positionierschale und den im Prüfkörper befindlichen Probenankern aus der Prüfkörperschalung.

14. Vorrichtung zur Herstellung eines Prüfkörpers aus einem erhärtendem Material mit darin eingebetteten Probenankern, die aufweist:
- eine Mehrzahl von Probenankern,
- eine Positionierschale, die eine Mehrzahl von Fixierpunkten aufweist, die zur Fixierung der Probenanker vorgesehen sind,
- eine Mehrzahl von Fixierelementen, die zur Fixierung der Probenanker an den Fixierpunkten der Positionierschale dienen,
- wobei die Positionierschale and jedem Fixierpunkt eine Versenkung aufweist, die ein Überstehen des Fixierelements verhindert, und
- wobei die Verbindung der Probenanker mit der Positionierschale gegenüber dem einzubringenden erhärtenden Material dicht ist.

15. Vorrichtung zur Herstellung eines Prüfkörpers aus einem erhärtendem Material mit darin eingebetteten Probenankern, die aufweist:
- eine Mehrzahl von Probenankern, die in der Probekörperschalung an der oberen, freien Oberfläche positioniert sind,
- mindestens eine Querhalterung, die eine Mehrzahl von Fixierpunkten aufweist, die zur Fixierung der Probenanker vorgesehen sind, die an der oberen freien Oberfläche positioniert sind,
- eine Mehrzahl von Fixierelementen, mit denen die die Mehrzahl von Probenanker an der mindestens einen Querhalterung befestigt werden,
- wobei die mindestens eine Querhalterung an der Probekörperschalung befestigt ist, und
- wobei Mehrzahl von Probenankern an den mindestens eine Querhalterung mit den Fixierelementen so befestigt sind, dass sie so anliegen, dass sie dicht gegen das einzubringende erhärtendem Material sind.
